# EUROPEAN PATENT SPECIFICATION

(11) **EP 2 508 885 B1**
(45) Date of publication and mention of the grant of the patent: **08.03.2017**
(21) Application number: 10833417.8
(22) Date of filing: 30.11.2010
(51) Int. Cl.: G01N 33/531, G01N 33/543

(54) **HOMOGENOUS MEASUREMENT METHOD AND MEASURING REAGENT**
HOMOGENES MESSVERFAHREN UND MESSREAGENS DAFÜR
PROCÉDÉ DE MESURE HOMOGÈNE ET RÉACTIF DE MESURE

(30) Priority: 30.11.2009 JP 2009272437
(43) Date of publication of application: 10.10.2012
(73) Proprietor: Sekisui Medical Co., Ltd., Tokyo 103-0027 (JP)
(72) Inventor: TAKAHASHI, Hiroshi, Ryugasaki-shi Ibaraki 301-0852 (JP); TAKAHASHI, Yuki, Ryugasaki-shi Ibaraki 301-0852 (JP); KANEKO, Chie, Ryugasaki-shi Ibaraki 301-0852 (JP)
(74) Representative: Hoffmann Eitle
(86) International application number: PCT/JP2010/071402
(87) International publication number: WO 2011/065573

(56) References cited:
- WO-A1-00/19201
- JP-A- 1 014 628
- JP-A- 7 301 632
- JP-A- 7 301 632
- JP-A- 9 068 529
- JP-A- 11 014 628
- JP-A- 2006 507 002
- JP-A- 2008 082 777
- NAOKI KOBAYASHI ET AL.: 'Zenjido Koso Men'eki Bunseki Sochi OLYDAS-120/Graozaim [New] Shiyaku ni yoru T3, T4, TSH Sokutei no Kisoteki Kento' JAPANESE JOURNAL OF CLINICAL LABORATORY AUTOMATION vol. 20, no. 5, 01 October 1995, pages 707 - 711
- KOJI ISHIDA ET AL.: 'Hitachi 736-60 no Trouble Kaiseki to Hoshu Kanri' JAPANESE JOURNAL OF CLINICAL LABORATORY AUTOMATION vol. 14, no. 2, 01 April 1989, pages 122 - 127
- YOSHIFUMI KOTAKE: 'Zenjido pH/Ketsueki Gas · Denkaishitsu - Glucose - Hematocrit Bunseki Sochi -Rapidpoint 400 no Shiyo Keiken to Hyoka' THE JOURNAL OF MEDICINE vol. 50, no. 2, 25 August 2003, pages 211 - 218

## Description

### TECHNICAL FIELD

The present invention relates to a measuring reagent for an automated analyzer characterized by inclusion of a silicone-based defoaming agent for reducing a matrix effect originating from the sample and for suppressing variability of measurement accuracy in different automated analyzers having different specifications, used in homogeneous latex agglutination immunoassay methods employing insoluble carrier particles, and in particular, in a latex agglutination immunoassay method employing insoluble carrier particles that support an antibody, an antigen, etc.

### BACKGROUND ART

In recent years, the amounts of the sample to be analyzed (hereinafter also referred to as "sample") and the test reagents required for performing automated analysis by automated analyzers in clinical tests are becoming smaller, thanks to the improved dispensing functions of the automated analyzers and the corresponding development of the clinical test reagents (hereinafter also referred to as "test reagents").

Among these clinical test reagents, the reagents for homogeneous measurement (assay) methods involving insoluble carrier particles, and for the latex agglutination immunoassay (LTIA) in particular, are finding wide applications to increasingly diverse targets due to the high sensitivity provided by them. However, there is still a need for improvement of these reagents in terms of making them more suitable for use in smaller amounts. There is also a need for improving said reagents in such a way that the same reagents can be used more universally in different automated analyzers having different specifications.

One of the challenges in attempting to use the test reagents in smaller amounts is to reduce the matrix effect caused by the samples such as serum taken from subjects. The matrix effect for example refers to a situation in which the quantity of a substance of interest measured in a sample (e.g. serum) taken from a subject appears larger or smaller than the quantity measured in a buffer solution containing the same substance in a purified form at the exactly same concentration and in the exactly same volume as said sample, said inconsistency being caused by the presence of some components (e.g. components other than the substance of interest) in said sample. In this Description, a sample such as serum collected from a subject is also referred to as a "biological sample".

The approaches that have been taken in order to reduce the matrix effect include dilution of the biological samples, as substances of interest are usually present in a relatively high abundance in the samples of conventional clinical tests, and inclusion of biological sample-derived components (e.g. serum or albumin) in the calibration standards (calibrators) and in the test reagents so that the biological samples subject to measurement and the calibration standards are more comparable in this respect. However, the method of reducing the matrix effect by dilution cannot be employed if the amount of the substance of interest in the biological sample becomes outside the sensitivity range of the assay system, and the addition of the serum component or the like to the calibration standards or the test reagents may cause an increase of viscosity or foaming in the reagents which may work against the intention of minimizing dispensing volumes.

A problem that needs to be overcome in order to realize universal usability of the same test reagents in a plurality of automated analyzers having different specifications is the fact that different models of automated analyzers can exhibit variability in accuracy and reproducibility of measurements (collectively referred to as "measurement accuracy"; lack of measurement accuracy may be referred to as "impaired performance") even if an identical test reagent is used (hereinafter, this general phenomenon is also referred to as "inter-model variability"). Such impaired performance may be found only when a particular reagent is applied to a particular automated analyzer, and thus the problem is hardly predictable when the test reagent is still in a product development stage (where it is not practical to conduct a performance test of the reagent with each and every model of automated analyzers available on the market). More typically, impaired performance of a test reagent becomes obvious only after medical or other facilities have started to use it, which is quite problematic.

Clinical test reagents have been conventionally provided with descriptions of recommended measurement conditions (parameters) suitable for each of the several models of automated analyzers in which the reagents are intended to be used, but varying parameters alone is sometimes not sufficient for improving the inter-model variability mentioned above. Another approach sometimes taken is to develop a plurality of test reagent formulas suitable for different models of automated analyzers in relation to a single item to be analyzed (test item). However, there are so many models of automated analyzers and therefore it is difficult in terms of workload and economical efficiency to develop individually suitable formulas of test reagents for specific models of automated analyzers.

Methods of reducing the matrix effect have not been hitherto investigated sufficiently in relation to homogenous assay reagents using insoluble carrier particles for automated analysis (LTIA reagents in particular). It is also not clearly understood what causes the performance differences between different models of automated analyzers or how to improve the problem.

LTIA reagents containing dextran sulfate (known to have a thickening effect) and albumin (known to have a foaming effect) and further containing a defoaming agent have been disclosed (Patent Documents 1 and 2). Patent Document 1 describes that an LTIA measurement was carried out by using a reagent containing 1.25 to 1.75% dextran sulfate sodium and 2.0% fatty-acid-free human serum albumin and formulated with 0.01% defoaming agent 1410 (manufactured by Dow Coming Corporation), and Patent Document 2 describes that an LTIA measurement was carried out by using a reagent containing 1% dextran sulfate sodium and 0.5% bovine serum albumin and formulated with 0.005 % defoaming agent (1410, manufactured by Dow Coming Corporation). However, both of these documents relate to a total liquid volume of 300 µL, which is larger than a normal total volume currently used in automated analyses at the time of the present application where the combined liquid volume of a sample and a reagent (test solution) is about 200 µL. Patent Documents 1 and 2 have not been written on the premise that the sample and reagent volumes are to be reduced. Moreover, in Patent Documents 1 and 2, no reference is made to possible involvement of the defoaming agent in a reduction of the matrix effect or what causes the inter-model variability or how to improve it. In this Description, if a reagent is in a liquid form, the reagent may be referred to as a reagent solution, or more simply, a test solution.

In various measurement methods that are based on the principle of binding assays that employ insoluble carrier particles supporting an antibody, an antigen or the like, a surfactant is often contained in some components of the assay reagents such as a washing solution or a reaction buffer solution for the purpose of suppressing nonspecific reactions. However, the presence of the surfactant naturally renders forming events more likely during stirring/mixing of a reaction solution, which would affect accuracy of the measurements. In this light, methods of suppressing surfactant-induced foaming by addition of a defoaming agent have been reported, as in the heterogeneous enzyme immunoassay described in Patent Document 3. In the reagents for amplifying and detecting polynucleotide described in Patent Document 4, addition of a defoaming agent is proposed in relation to the application in microfluid devices characterized by micrometer-size narrow channels for flowing the reagents (test solutions).

In relation to automated analyzers, it has been proposed, for example in Patent Document 5, to eliminate air bubbles caused by a surfactant contained in an agent for washing the automated analyzer by adding thereto a defoaming agent. However this proposal concerns solving a problem in washing procedures (in other words, maintenance procedures) of the equipment, and it is not intended to be used in the measurements of the samples. As such, Patent Document 5 of course does not mention any possible effect on measurement accuracy. Moreover, a homogenous measurement method does not require a washing procedure in the first place and therefore under no circumstances the reaction could become contaminated with a detergent due to a washing procedure.

Patent Document 6 relates to a non-homogeneous measurement method requiring bound/free separation, including an anti-foaming agent to avoid the presence of foam when small samples are analysed, to ensure proper pipetting volumes. Such an anti-foaming agent must not interfere with or alter the result of the assay, that is, the results of the assay obtained with and without the anti-foaming agent must not be significantly different.

As can be seen from Patent Documents 1 to 5, a defoaming agent has not hitherto been used for the purpose of reducing the matrix effect or improving the inter-model variability of automated analyzers in a homogeneous measurement method reagent comprising insoluble carrier particles, or more particularly, in an LTIA reagent.

### PRIOR ART DOCUMENTS

### PATENT DOCUMENTS

Patent Document 1: JP H07-301632 A
Patent Document 2: JP H11-014628 A
Patent Document 3: JP H09-068529 A
Patent Document 4: JP 2006-507002 A
Patent Document 5: JP 2008-82777 A
Patent Document 6: WO 00/19201 A1

### SUMMARY OF THE INVENTION

### PROBLEM TO BE SOLVED BY THE INVENTION

An objective of the present invention is to provide a measurement method and a measuring reagent for an automated analyzer wherein a matrix effect originating from a biological sample is reduced and variability in measurement accuracy among different automated analyzers having different specifications is suppressed, said method being a homogeneous latex agglutination immunoassay method employing insoluble carrier particles, or more particularly a latex agglutination immunoassay method employing insoluble carrier particles that support an antibody, an antigen, etc..

### MEANS FOR SOLVING THE PROBLEM

The present inventors have conducted a wide range of investigations to attain said objective. As a result, the inventors have discovered that addition of a silicone-based defoaming agent to the measuring reagent can reduce a matrix effect originating from a biological sample and suppress variability in measurement accuracy among different automatic analyzers having different specifications without affecting the basic performance of said reagent. This discovery has led to the completion of the measuring reagent of the present invention.

The present invention comprises the following.
(1) A measuring reagent for a homogeneous latex agglutination immunoassay method for an automated analyzer comprising latex particles, characterized in that
   the measuring reagent comprises more than one constituent reagents,
   one of the constituent reagents contains the latex particles,
   the latex particles support a substance that binds an analyte with a high affinity, the substance being selected from a polyclonal antibody, a monoclonal antibody, a recombinant antibody, a functional fragment of the previously mentioned antibodies, or a natural or recombinant antigen,
   another constituent reagent contains a silicone-based defoaming agent, wherein a concentration of the silicone-based defoaming agent in said another constituent reagent is 0.0001 to 0.1%,
   the protein concentration of each constituent reagent is less than 2% (w/v), and said measuring reagent is used for a measurement wherein a total liquid volume of a sample and said measuring reagent dispensed by the automated analyzer is less than 300 µL, a liquid volume of said constituent reagent comprising the latex particles accounts for 20 to 50% (v/v) relative to the total liquid volume,
   wherein the protein concentrations of the constituent reagents
   do not include proteins supported on the carrier particles as the substance that binds the analyte with high affinity, or blocking proteins that coat the carrier particles.
(2) The reagent according to (1), wherein the silicone-based defoaming agent is of a type selected from the group consisting of oil, oil compound, solution, self-emulsion, emulsion, and a mixture thereof.
(3) A homogeneous latex agglutination immunoassay method using an automated analyzer comprising the steps of:
   1) dispersing a measuring reagent and a sample containing an analyte, wherein
      the measuring reagent comprises more than one constituent reagents;
      one of the constituent reagents contains latex particles;
      the latex particles support a substance that binds an analyte with a high affinity, the substance being selected from a polyclonal antibody, a monoclonal antibody, a recombinant antibody, a functional fragment of the previously mentioned antibodies, or a natural or recombinant antigen;
      another constituent reagent contains a silicone-based defoaming agent, wherein a concentration of the silicone-based defoaming agent in said another constituent reagent is 0.0001 to 0.1%; and
      the protein concentration in each constituent reagent is less than 2% (w/v);
   2) mixing the sample and the measuring reagent in such a way that a total liquid volume of the sample and the measuring reagent dispensed by the automated analyzer is less than 300 µL, said constituent reagent containing the latex particles accounts for 20 to 50% (v/v) relative to the total liquid volume, the final protein concentration during the measurement in the automated analyzer is less than 1% (w/v) relative to the total liquid volume, wherein the protein concentrations of the constituent reagents and the final protein concentration do not include proteins supported on the carrier particles as the substance that binds the analyte with high affinity, or blocking proteins that coat the carrier particles; and
   3) detecting the analyte.

### EFFECT OF THE INVENTION

The present invention makes it possible to provide an agglutination measuring reagent for an automated analyzer that is based on a homogeneous LTIA method employing insoluble carrier particles, said reagent being capable of high-accuracy measurements without being affected by the matrix effect originating from a biological sample or by different specifications of the automated analyzers.

### BRIEF DESCRIPTION OF DRAWING

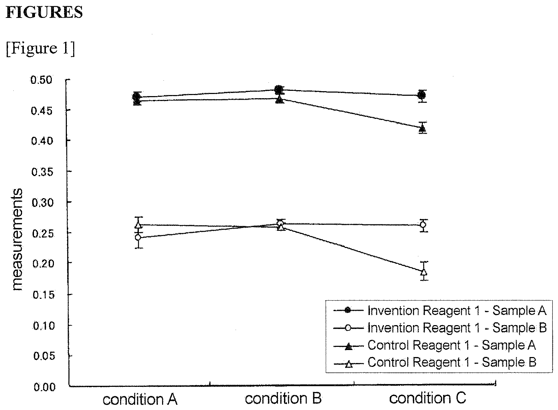

[Fig. 1] The graph shows variability of measurement values in five sequential measurements of each of the serum samples A and B obtained with three different parameter settings (conditions A to C) and by using Control Reagent 1 and Invention Reagent 1 (Example 2).

### MODES FOR CARRYING OUT THE INVENTION

### (Homogeneous Measurement Method)

In the present invention, a homogeneous measurement method (or a homogeneous assay method) refers to a measurement method of specifically detecting an ongoing reaction that involves an analyte in a mixture (reaction solution) of a sample and a reagent without performing B/F (bound/free) separation, which is distinguished from a heterogeneous measurement method in which a main reaction is allowed to continue and be detected after excess components that have not been involved in the reaction are completely removed and washed off by a B/F separation step.

### (Automated Analyzer)

In the present invention, an automated analyzer refers to those manufactured/sold by companies mainly for use in clinical tests. Specific examples include general reagent type automated analyzers such as the automated analyzer series manufactured by Hitachi High-Technologies Corporation, the TBA series manufactured by Toshiba Medical Systems Corporation, the BM series manufactured by JEOL Ltd., and those manufactured by Beckman Coulter Biomedical Ltd., Sekisui Medical Co., Ltd., and the like, as well as specific reagent type automated analyzers such as the near-infrared measuring instrument LPIA (registered trademark) (manufactured by Mitsubishi Chemical Medience Corporation) and the scattered light intensity measuring instrument (manufactured by Dade Behring Inc.), and the blood coagulation measuring instruments capable of performing optical measurements. The instrument may be a large- or small-scale machine and may be called by different brand names.

Sample measurements by these automated analyzers are typically carried out in the manner described below. Each step of a measurement will be described in relation to an exemplary embodiment in which the test reagent consists of two constituent reagent solutions which is a preferable embodiment (two-reagent system). First, aliquots of a sample and a first reagent are sequentially taken up and dispensed into reaction vessels (which are also measurement cells in which absorbance measurements will be made) and mixed. Next, a second reagent is taken up and dispensed into the reaction vessels and mixed, and then, optical changes occurring within a certain time period is measured. Many automated analyzers on the market for use in clinical tests are capable of providing functions/specifications that are needed for performing the steps described above.

However, specific details of the functions or specifications for individual steps (such as dispensing of the sample and the reagents and stirring and mixing of the solutions) may be different between different models of automated analyzers. For example, increasingly diverse approaches for the stirring/mixing step are becoming available, concurrently with the minimization trend of the sample and reagent volumes required for a measurement. Examples of the new approaches include direct modes (contact modes) such as a system in which reaction solutions are stirred and mixed by rotating probes of various shapes (e.g. HITACHI 7180 manufactured by Hitachi High-Technologies Corporation) and a system utilizing the vibrations generated by piezoelectric element vibrating probes to mix reaction solutions (e.g. TBA120FR manufactured by Toshiba Medical Systems Corporation), as well as non-contact modes (indirect modes) such as a system of vibrating a reaction solution in a reaction vessel by ultrasonic waves to mix the reaction solution (e.g. HITACHI 9000 manufactured by Hitachi High-Technologies Corporation), a system in which mixing of a reaction solution is carried out by the force generated while the sample and reagent solutions are being discharged from a probe designed for dispensing these solutions (e.g. CP2000 manufactured by Sekisui Medical Co., Ltd.), and a system in which a reaction solution is mixed by shaking the reaction vessel itself (e.g. CP2000 manufactured by Sekisui Medical Co., Ltd.). As used herein, the term stirring/mixing referring to a function of an automated analyzer is intended to mean either the automated analyzer has only a "stirring" function, or a "mixing" function, or both functions.

Since these different systems of stirring/mixing are based upon fundamentally different principles, it is conceivable that the stirring/mixing abilities provided by them may vary between each other, and that unevenness of reactions caused by different stirring/mixing abilities may be becoming prevalent in clinical tests that use newer automated analyzers. Moreover, different solution-dispensing mechanisms and different materials used for individual parts of the automated analyzer instruments, although not easily comparable in a direct way, are believed to give entirely different physical influences on the test reagents. The present invention is preferably used where there is inter-model variability of automated analyzers that is possibly caused by different specifications of stirring/mixing functions.

### (Silicone-Based Defoaming Agent)

Foam (or bubble) formation is a phenomenon that occurs in an interface between a gas phase and a liquid phase. Air bubbles are generated when air is trapped inside thin films of liquid. Air bubble formation is influenced by surface tension, viscosity, and so on, and known factors for causing air bubbles include surfactants and high-molecular-weight compounds. It is believed that surfactant molecules are regularly arranged on the surface of air bubbles with their hydrophobic groups bordering on the gas phase. In the present invention, the term "defoaming" refers to a foam-suppressing effect in which air bubble formation is suppressed by interfering with generation and maintenance of the regularly arranged structures of the surfactant, and a bubble-bursting effect in which air bubbles are broken, as well as a deaeration effect in which air bubbles are conglomerated and lifted up to the surface of the liquid. The silicone-based defoaming agent used in the present invention may have any one of the effects mentioned above, although an agent having two or more of the above effects is preferable.

Type of the silicone-based defoaming agent in the measuring reagent of the present invention is not particularly limited as long as the agent is compatible with the homogeneous measurement method. Examples of the silicone-based defoaming agents include those comprising polyalkylsiloxane.

Polyalkylsiloxane that may be used in the present invention has the structure expressed by the following Chemical Formula 1. 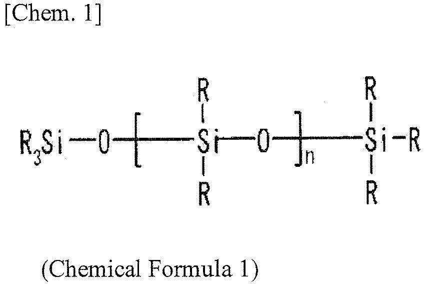 In this formula, R may be a functional group such as a hydrogen atom, an alkyl group, a substituted alkyl group, and an aromatic group, and more specific examples of R include the groups shown in the following Chemical Formula 2.

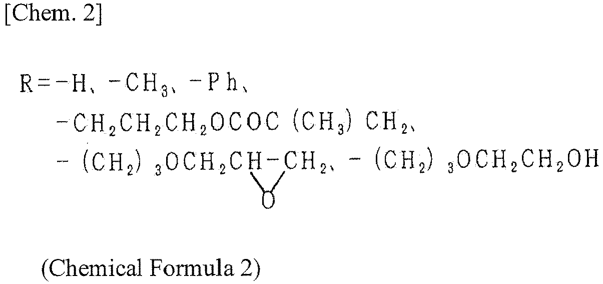

R is more preferably a methyl group or a phenyl group. In Chemical Formula 1, all R groups may be identical or R groups may comprise two or more different kinds of groups. If R groups comprise two or more different kinds of groups, such polyalkylsiloxane may be a homopolymer or a block copolymer expressed by the following Chemical Formula 3.

In this embodiment, the extent of polymerization, i.e., the value of "n" in Chemical Formula 1 or "n+m" in Chemical formula 3, may be 50 or lower and, preferably, the extent of polymerization is 1 to 20. If polymerization is more extensive than these ranges, viscosity at room temperature may become too high and uniform dispersion may become more difficult.

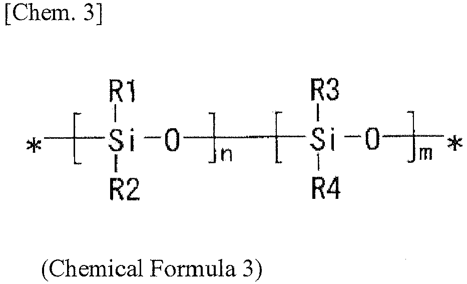

Specific examples of polyalkylsiloxanes described above include dimethylpolysiloxane, diphenylpolysiloxane, polymethylphenylsiloxane, polymethylepoxypropylsiloxane and others wherein the extent of polymerization is 1 to 20.

Examples of commercially available polyalkylsiloxanes include TSF451, THF450, FQF501, YSA6403, TSA720, YSA02, TSA750, TSA750S, YSA6406, TSA780, TSA7341, TSA739, TSA732, TSA732A, TSA772, TSA730, TSA770, TSA775, YMA6509, TSA737B, TSA737S, and TSA737F (all manufactured by GE Toshiba Silicone); KM-73, KM-73A, KM-73E, KM-70, KM-71, KM-75, KM-85, KM-72, KM-72F, KM-72S, KM-72FS, KM-89, KM-90, KM-98, KM-68-1F, KS-508, KS-530, KS-531, KS-537, KS-538, KS-66, KS-69, KF-96, KS-604, KS-6702, FA-630, KS-602A, KS-603, FA-600, KM-88P, KM-91P, and KM-601S (all manufactured by Shin-Etsu Silicone); and SH200, SH203, FS1265, SH5500, SC5540, BY28-503, SH7PA, SH5510, SH5561, SH5507, SH8730, SM5511, SM5571, SM5515, SM5512, DC200, FS1265, DC71, DC74, DB-100, F-16, DC75, 1266, 1283, DKQ1-1183, DKQ1-1086, DKQ1-071, 80, 544, EPL, 025, 1224, 1233, DKQ1-1247, 013A, 1277, CE, C-Emulsion, AFE, 92, 93, DB-110N, and DC2-4248S (all manufactured by Dow Coming Toray Co., Ltd.). The diverse polyalkylsiloxanes described above may be used individually or as a mixture of two or more types.

The silicone-based defoaming agents that can be added to the measuring reagents of the invention include those selected on the basis of their defoaming effects from the group consisting of: a modified silicone formed by introducing a reactive group into dimethylpolysiloxane; a silicone surfactant having a surfactant-like structure comprising a hydrophobic group consisting of methylpolysiloxane and a hydrophilic group consisting of polyalkylene oxide; and a silicone resin. A mixture of two or more of the above may also be comprised in the silicone-based defoaming agent of the present invention.

The silicone products that may be used as a silicone-based defoaming agent of the present invention may be any of the various types classified according to their chemical forms or properties, such as oil, oil compound, solution, self-emulsion, emulsion, and the like. In general, the oil type refers to a silicone oil that is used by itself, and the solution type refers to a silicone oil diluted in an organic solvent. The compound type refers to a silicon oil containing fine powder of silica or the like, and the emulsion type refers to a silicone oil emulsified by a nonionic surfactant or the like. The self-emulsion type refers to a silicone oil comprising an alkyleneoxy group or the like within its structure and may also include what is called modified silicon oils. The powder type refers to a silicone oil absorbed on oil-absorptive powder. Among these types, the silicone-based defoaming agents of the self-emulsion type and the emulsion type tend to be readily and stably dispersed in the measuring reagents of the invention by forming emulsion therewith, and are preferably used. As used herein, the terms "silicone" and "silicone oil" are used in the conventional senses unless otherwise noted.

Examples of compositions of the silicone-based defoaming agents of the present invention include dimethylsilicone, modified silicone, silicone oil + solvent, silicone oil + silica, water-soluble silicone/water-soluble organic substance, silicone compound/emulsifier/water, etc.

Silicone products that may be used as a silicone-based defoaming agent in the measuring reagent of the present invention are commercially available from, for example, Momentive Performance Materials Japan LLC., Dow Coming Toray Co., Ltd., Shin-Etsu Chemical Co., Ltd., and BYK Japan KK (it should be noted that manufacturing companies and sales companies are not strictly distinguished in the present Description). Most suitable ones may be selected from these diverse silicone products capable of providing a defoaming effect, by checking their influence (or lack thereof) on the main reaction to be measured and stability of the resulting reagents. Silicone is sometimes referred to as silicon.

Among the commercially available silicone-based defoaming agents described above, preferable examples include YSA6406 (a self-emulsifying oil compound type silicone-based defoaming agent: it contains alkyl-modified silicone oil, polyether-modified silicone, silica, emulsifier, and others), TSA7341 (an emulsion type defoaming agent: it contains polyalkylsiloxane, silica, and others), and TSA775 (an emulsion type defoaming agent: it contains polyalkylpolysiloxane, polyether-modified silicone oil, silica, emulsifier, and others) that can be obtained from GE Toshiba Silicone or Momentive Performance Materials Japan LLC.

The silicone products mentioned above are usually categorized by different purposes such as industrial use and food additive use. However, there has been no "clinical test reagent" category, and thus there are no known standards for such application. Thus, in the present invention, it is desirable to first check for any changes in the measurement sensitivity upon addition of various silicone-based defoaming agents to the measuring reagent of interest, and to select ones that show little effect on the reaction itself to be measured.

There is no limitation to the amount of the silicone-based defoaming agent added in the measuring reagent of the present invention as long as the main reaction (such as an antigen-antibody reaction) of interest is not strongly affected and the stability of the test solution is not adversely affected. A preferable concentration of the silicone-based defoaming agent can also be empirically determined based on observed defoaming effects or other criteria. A defoaming effect can easily be checked, for example, by verifying absence of persistent air bubbles on the solution surface or vessel walls when the test solution is shaken vigorously, or by verifying suppression of air bubbles in an easily-foaming solution. The concentration of the silicone-based defoaming agent is 0.0001 to 0.1 % and preferably 0.001 to 0.01 % in the constituent reagent.

### (Agglutination Measuring Reagent)

If the insoluble carrier in the measuring reagent of the present invention supports a substance that binds the analyte with a high affinity, the reagent is particularly referred to as an agglutination measuring reagent. In the agglutination measuring reagent of the present invention, examples of the substances that may be supported on the insoluble carrier particles include proteins, peptides, amino acids, lipids, carbohydrates, nucleic acids, and haptens. There is no particular limitation as to whether the molecular weight of the substance is high or low or whether the substance is naturally-derived or synthetically-derived. However, in the agglutination method, the extent of agglutination increases in proportion to the concentration of the analyte. The high-affinity-binding substance employed is a polyclonal antibody, a monoclonal antibody (including a recombinant antibody and a functional fragment of each antibody), or a natural or recombinant antigen.

### (Insoluble Carrier Particles)

Types of materials that may be used as insoluble carrier particles in the measuring reagent of the present invention are not particularly limited as long as the material is compatible with the purpose of the test reagent, but specific examples include latex, metal colloid, silica, and carbon. The size of the insoluble carrier particles may be selected as needed in the range of 0.05 to 1 µm depending on the detection principle used by the particle agglutination measurement method and the reagent of the present invention. An average particle diameter used in an optical measurement in an automated analyzer is generally 0.1 to 0.4 µm and preferably 0.1 to 0.2 µm. An average particle diameter of the insoluble carrier particles can be checked by a particle size analyzer, transmission electron microscope imaging, or other methods.

### (Other Reagent Components of Homogeneous Measurement Method)

In addition to the main components for the reaction, the homogeneous measuring reagent of the present invention may contain a component for buffering the pH, ionic strength, osmotic pressure, etc. of the sample, such as acetic acid, citric acid, phosphoric acid, tris, glycine, boric acid, carbonic acid, and Good's buffer as well as sodium salts, potassium salts, and calcium salts thereof and inorganic salts such as NaCl and KCl. The homogeneous measuring reagent may further contain a component for enhancing the agglutination of the insoluble carrier particles, such as macromolecules including polyethyleneglycol, polyvinylpyrrolidone, and phospholipid polymers. The homogeneous measuring reagent may also contain one or more of components for controlling agglutination, such as proteins, amino acids, carbohydrates, metal salts, surfactants, reducing agents, and chaotropic agents that are generally used for this purpose. Any components that tend to cause foaming may also be added to the measuring reagents of the present invention. In any case, the concentration of the protein added to each constituent reagent (which together makes up the measuring reagent of the present invention) is less than 2% (w/v), and the final concentration during the measurement in the automated analyzer (i.e. the concentration in the total liquid volume consisting of the sample and the reagent) is less than 1% (w/v).

The references to the protein concentrations made above only relate to the components outside the main components of the reaction. Thus, the concentrations mentioned above do not include the proteins supported on the insoluble carrier particles (the substances that bind the analytes with a high affinity) or the blocking proteins that coat the carrier particles.

### (Sample Subjected to Measurement and Analyte)

The type of the sample to be measured (assayed) with the agglutination measuring reagent of the present invention is not particularly limited, and may be any one of a variety of biological samples. Preferable examples include biological fluids such as blood, serum, plasma, and urine. The analyte (i.e. the substance of interest) can be protein, peptide, amino acid, lipid, carbohydrate, nucleic acid, or hapten, for example, or any other molecules that are quantifiable in theory. Examples of the analytes include CRP (C-reactive protein), Lp(a), MMP3 (matrix metalloproteinase 3), anti-CCP (cyclic citrullinated peptide) antibody, anti-phospholipid antibody, RPR, type IV collagen, PSA, BNP (brain natriuretic peptide), NT-proBNP, insulin, microalbumin, cystatin C, RF (rheumatoid factor), CA-RF, KL-6, PIVKA-II, FDP, D-dimer, SF (soluble fibrin), TAT (thrombin-antithrombin III complex), PIC, PAI, factor XIII, pepsinogen I/II, phenytoin, phenobarbital, carbamazepine, valproic acid, theophylline, and others.

### (Configuration and Usage of Measuring Reagent)

The measuring reagent for an automated analyzer of the present invention is made up of more than one constituent reagents. One of the constituent reagents contains the insoluble carrier particles described above and another constituent reagent contains the silicone-based defoaming agent. The defoaming effect can easily be checked by mixing said constituent solutions containing the silicone-based defoaming agent and other constituent solutions at the same ratio as in an actual measurement, shaking the mixture vigorously, and verifying absence of persistent air bubbles on a solution surface and vessel walls. As mentioned above, the protein concentration in each of the constituent reagents making up the measuring reagent of the present invention is less than 2% (w/v), and the final concentration at the time of measurement in an automated analyzer (in the total volume of the sample and the reagent solutions) is designed to be less than 1% (w/v).

### (Usage of Measuring Reagent)

If the measuring reagent of the present invention consists of two constituent reagents, namely a first reagent and a second reagent, and the insoluble carrier particles are contained in either one of the reagents, the volume ratio of the first reagent and the second reagent used in the measurement should preferably be 4:1 to 1:1. The total liquid volume of a sample and reagent solutions dispensed by the automated analyzer is less than 300 µL, and the reagent solution containing the insoluble carrier particles constitutes 20 to 50% (v/v) of the total liquid volume. The concentration of the insoluble carrier particles supporting a high-affinity-binding substance for the analyte is 0.05 to 0.3% (w/v) at the time of measurement in the automated analyzer (relative to the total volume of the sample and the reagent solutions).

### EXAMPLES

The present invention will be described in detail by referring to the examples below, but the present invention is not limited to these examples.

### [Example 1] Reduction of Matrix Effect

Reduction of the matrix effect by the present invention was verified.
(1) Reagent: SS Type Pure Auto (registered trademark) S, CRP Latex (manufactured by Sekisui Medical Co., Ltd.)

### (1-1) First Reagent

(i) Control Reagent 1: SS Type Pure Auto (registered trademark) S, CRP Latex, Buffer Solution 1
(ii) Invention Reagent 1: Silicone-based defoaming agent YSA6406 (manufactured by Momentive Performance Materials Japan LLC.) was added to Control Reagent 1 at a final concentration of 0.001 % to provide Invention Reagent 1.

### (1-2) Second Reagent

SS Type Pure Auto (registered trademark) S, CRP Latex, Latex Solution 2. The protein concentration in Second Reagent is about 0.3% (w/v).

### (1-3) Calibrator

### SS Type Pure Auto (registered trademark) S, CRP Latex, Calibrator

### (2) Automated Analyzer: TBA120FR (manufactured by Toshiba Medical Systems Corporation)

### Parameter Conditions

(i) Liquid volume: Sample - First Reagent (Control Reagent 1 or Invention Reagent 1) - Second Reagent
   Condition A: 3 µL - 210 µL - 70 µL
   Condition B: 2.5 µL - 175 µL - 58 µL
   Condition C: 2 µL - 140 µL - 47 µL
(ii) Analysis method: rate method (photometric points 19-28)
(iii) Measurement wavelength: 604 nm
(iv) Calibration: spline

### (3) Samples Subjected to Measurements

Serum Sample: CRP concentration 0.5 mg/dL
Mock Sample: 20 mM Tris-HCl Buffer Solution (pH 7.5) containing CRP at a concentration 0.5 mg/dL and 0.1 % BSA, but no serum components (4) Measurement Method

Each of the two samples (the serum sample and the mock sample) was subjected to five sequential measurements by using each of the two types of First Reagents (Control Reagent 1 and Invention Reagent 1) and three different parameters (Conditions A to C) in which only the total reaction volumes were varied while the volume ratios of the sample and the reagents were fixed. Averages and coefficients of variation were calculated to verify accuracy and reproducibility.

The results of the measurements are shown in Table 1. When measurement accuracies obtained with Control Reagent 1 against the two samples were compared, differences in the measurement reproducibility between the serum sample and the mock sample were observed under each of Conditions A to C. Measurement reproducibility with the mock sample not containing serum components was very poor, as indicated by the coefficient of variation of about 5%. Measurement reproducibility with the serum sample was good under all conditions, but under Condition C in which the total volume is reduced, the values measured from the serum sample was smaller by a margin of 10% or more compared with the mock sample measurements, which suggested rather poor accuracy. From the above results, it was believed that the measurement accuracy in Control Reagent 1 was significantly affected by the matrix components originating from the sample. On the other hand, when measurement accuracies obtained with Invention Reagent 1 against the two samples were compared, the accuracy and the reproducibility of the measurements were virtually consistent under all of Conditions A to C regardless of the sample type. The discrepancies observed between the serum sample and the mock sample (lacking serum components) were reduced compared to the measurements made with Control Reagent 1. In particular, the discrepancy of the measurement values between the serum sample and the mock sample under Condition C was reduced to 3%, demonstrating a significant improvement provided by Invention Reagent 1. In summary, with Control Reagent 1, not only reproducibility of the reaction but also (depending on the total volume) soundness of the measurement itself were adversely affected, suggesting a significant influence of the matrix effect. On the other hand, with Invention Reagent 1, consistent performance was maintained regardless of the components contained in the samples.

**[Table 1]**

| Control Reagent 1 | | | | | | |
|---|---|---|---|---|---|---|
| sample-reagents volumes | A:3-210-70 | | B:2.5-175-58 | | C:2-140-47 | |
| sample | serum | mock | serum | mock | serum | mock |
| five sequential measurements (mg/dL) | 0.456 | 0.518 | 0.464 | 0.466 | 0.407 | 0.504 |
| | 0.477 | 0.538 | 0.460 | 0.500 | 0.419 | 0.491 |
| | 0.460 | 0.471 | 0.475 | 0.471 | 0.415 | 0.447 |
| | 0.461 | 0.475 | 0.472 | 0.447 | 0.426 | 0.455 |
| | 0.467 | 0.487 | 0.460 | 0.449 | 0.418 | 0.469 |
| average | 0.464 | 0.498 | 0.466 | 0.467 | 0.417 | 0.473 |
| standard deviation | 0.008 | 0.029 | 0.007 | 0.021 | 0.007 | 0.024 |
| coefficient of variation V (%) | 1.76 | 5.84 | 1.49 | 4.58 | 1.65 | 5.07 |
| | | | | | | |

| Invention Reagent 1 | | | | | | |
|---|---|---|---|---|---|---|
| sample-reagents volumes | A:3-210-70 | | B:2.5-175-58 | | C:2-140-47 | |
| sample | serum | mock | serum | mock | serum | mock |
| five sequential measurements (mg/dL) | 0.477 | 0.471 | 0.441 | 0.448 | 0.476 | 0.464 |
| | 0.483 | 0.444 | 0.444 | 0.469 | 0.478 | 0.453 |
| | 0.457 | 0.458 | 0.440 | 0.451 | 0.468 | 0.453 |
| | 0.465 | 0.479 | 0.434 | 0.460 | 0.458 | 0.463 |
| | 0.468 | 0.462 | 0.439 | 0.462 | 0.465 | 0.450 |
| average | 0.470 | 0.463 | 0.440 | 0.458 | 0.469 | 0.457 |
| standard deviation | 0.010 | 0.013 | 0.004 | 0.009 | 0.008 | 0.006 |
| coefficient of variation (%) | 2.17 | 2.87 | 0.83 | 1.86 | 1.75 | 1.41 |

### [Example 2] Reduction of the Effect of Different Total Liquid Volumes

Improvement of the matrix effect by the present invention was verified.

### (1) Reagents and Automated Analyzer

The same first reagents ((i) Control Reagent 1 and (ii) Invention Reagent 1), second reagent, calibrator, automated analyzer, and parameter conditions described in Example 1 were used except that Serum Samples A and B were used as measurement samples.

Among the parameter conditions, only the liquid volumes are shown again below.

### (i) Liquid volume: Sample - First Reagent (Control Reagent 1 or Invention Reagent 1) - Second Reagent

Condition A: 3 µL - 210 µL - 70 µL (total liquid volume: 283 µL)
Condition B: 2.5 µL - 175 µL - 58 µL (total liquid volume: 235.5 µL)
Condition C: 2 µL - 140 µL - 47 µL (total liquid volume: 189 µL)

### (2) Measurement Method

Each of the serum samples A and B was measured five times sequentially by using the two types of first reagents (Control Reagent 1 and Invention Reagent 1) and the three different parameters (Conditions A to C) in which only the total reaction volumes were varied while the volume ratios of the sample and the reagents were fixed.

The results of the measurements are shown in Fig. 1. With Control Reagent 1, for each sample, the measured values changed (decreased in the present Example) as the total liquid volume was reduced, suggesting that Control Reagent 1 is affected by the differences in the total reaction volumes. On the other hand, with Invention Reagent 1, the measured values obtained from either of the serum samples A and B were substantially constant in all of the condition A to C. Therefore, it was confirmed that, while Control Reagent 1 requires a total volume above a certain level for providing accurate measurement values, Invention Reagent 1 is capable of providing accurate measurement values regardless of the total volume conditions.

### [Example 3] Reduction of the Effect of Different Stirring Mechanisms of Automated Analyzers (1)

SS Type Pure Auto (registered trademark) S, CRP Latex (manufactured by Sekisui Medical Co., Ltd.) was used in the measurement by 9000-series HITACHI automated analyzer (manufactured by Hitachi High-Technologies Corporation, equipped with a stirring/mixing function that performs mixing of the solutions in the reaction vessel by vibrating the solutions with ultrasonic waves) to confirm improvement of measurement accuracy achieved by the present invention.

### (1) Reagent: SS Type Pure Auto (registered trademark) S, CRP Latex

### (1-1) First Reagent

### (i) Control Reagent 1

Control Reagent 1 was as described in Example 1.

### (ii) Invention Reagents

Invention Reagents 1 and 2 were prepared by adding the silicone-based defoaming agents YSA6406 and TSA7341 (manufactured by Momentive Performance Materials Japan LLC.), respectively, to Control Reagent 1 at a final concentration of 0.001 %.

### (1-2) Second Reagent

Second Reagent was as described in Example 1.

### (1-3) Calibrator

The calibrator was as described in Example 1.

### (2) Automated Analyzer: HITACHI Automated Analyzer 9000

This instrument stirs and mixes the solutions in the reaction vessel by a non-contact mode employing vibrations created by ultrasonic waves.

### Parameter Conditions

(i) Liquid volumes: Sample - First Reagent - Second Reagent: 3 µL - 150 µL - 50 µL
(ii) Analysis method: two-point end method (photometric point 38-70)
(iii) Measurement wavelength: main wavelength 570 nm/ secondary-wavelength 800 nm
(iv) Calibration: spline

### (3) Samples Subjected to Measurements

Serum Samples 1 and 2

### (4) Measurement Method

Control Reagent 1 or Invention Reagent 1 or 2 was used as the first reagent and reproducibility within five sequential measurements in the 9000-series HITACHI automated analyzer was compared.

As shown in Table 2, with Control Reagent, the measurement reproducibility for Serum Samples 1 and 2 expressed as coefficient of variation (%) was 3.90 and 1.43, respectively, but it was 1.73 and 0.68 with Invention Reagent 1, and 2.52 and 1.54 with Invention Reagent 2. Thus, improvement of reproducibility was recognized in each case.

**[Table 2]**

| Reproducibility in five sequential measurements with Invention Reagents and Control Reagent | | | | | | |
|---|---|---|---|---|---|---|
| | reproducibility in measurement of Sample 1 | | | reproducibility in measurement of Sample 2 | | |
| | Inv. Reagent 1 0.001% YSA6406 | Inv. Reagent 2 0.001% TSA7341 | Ctrl. Reagent (defoaming component not added) | Inv. Reagent 1 0.001% YSA6406 | Inv. Reagent 2 0.001% TSA7341 | Ctrl. Reagent (defoaming component not added) |
| five sequential measurements (mg/dL) | 0.474 | 0.429 | 0.499 | 1.944 | 2.049 | 2.002 |
| | 0.471 | 0.452 | 0.484 | 1.966 | 2.049 | 1.937 |
| | 0.468 | 0.431 | 0.452 | 1.964 | 2.024 | 1.939 |
| | 0.482 | 0.429 | 0.490 | 1.969 | 2.046 | 1.966 |
| | 0.488 | 0.447 | 0.496 | 1.981 | 2.035 | 1.983 |
| average | 0.477 | 0.438 | 0.484 | 1.965 | 2.041 | 1.965 |
| standard dev. | 0.008 | 0.011 | 0.019 | 0.013 | | 0.028 |
| coefficient of variation (%) | 1.73 | 2.52 | 3.90 | 0.68 | 0.54 | 1.43 |

### [Example 4] Reduction of the Effect of Different Stirring Mechanisms of Automated Analyzers (2)

Invention Reagents 1 and 2 described in Example 3 were used in the measurement by Coapresta 2000 automated analyzer (manufactured by Sekisui Medical Co., Ltd., equipped with stirring/mixing functions that perform mixing of the solutions in the reaction vessel by using the force generated while the sample and reagent solutions are being discharged from a probe designed for dispensing these solutions and by shaking the reaction vessel itself) to confirm improvement of measurement accuracy achieved by the present invention.
(1) Reagents: as described in Example 1.
(2) Automated Analyzer: Coapresta 2000

A stirring/mixing mode in which the reaction vessel was shaken directly was used.

### Parameter Conditions

(i) Liquid volumes: Sample - First Reagent - Second Reagent: 3 µL - 150 µL - 50 µL
(ii) Analysis method: end method (photometric point 4-33)
(iii) Measurement wavelength: 570 nm
(iv) Calibration: spline
   (3) Measurement Samples: as described in Example 3.
   (4) Measurement Method

Invention Reagents 1 and 2 and Control Reagent were used and reproducibility within five sequential measurements of each sample in Coapresta 2000 was compared.

As shown in Table 3, the measurement reproducibility for Serum Samples 1 and 2 expressed as coefficient of variation (%) was 1.47 and 1.19 with Control Reagent, respectively, but 0.92 and 0.84 with Invention Reagent 1 and 0.67 and 1.17 with Invention Reagent 2. Thus, improvement of reproducibility was recognized in each case.

The results above confirm that measurement accuracy is improved by the use of the reagents of the present invention in different automated analyzers having different specifications with respect to stirring/mixing functions.

**[Table 3]**

| Reproducibility in five sequential measurements with Invention Reagents and Control Reagent | | | | | | |
|---|---|---|---|---|---|---|
| | reproducibility in measurement of Sample 1 | | | reproducibility in measurement of Sample of Sample 2 | | |
| | Inv. Reagent 1 0.001% YSA6406 | Inv. Reagent 2 0,001% TSA7341 | Ctrl. Reagent (defoaming component not added) | Inv. Reagent 1 0.001% YSA6406 | Inv. Reagent 2 0.001% TSA7341 | Ctrl. Reagent (defoaming component not added) |
| five sequential measurements (mg/dL) | 0.495 | 0.516 | 0.515 | 1.998 | 1.984 | 2.002 |
| | 0.501 | 0.511 | 0.506 | 1.973 | 1.953 | 1.982 |
| | 0.507 | 0.511 | 0.496 | 1.976 | 1.941 | 1.972 |
| | 0.499 | 0.513 | 0.500 | 1.951 | 1.991 | 1.961 |
| | 0.497 | 0.519 | 0.500 | 1.973 | 1.989 | 1.939 |
| average | 0.500 | 0.514 | 0.503 | 1.974 | 1.972 | 1.971 |
| standard dev. | 0.005 | 0.003 | 0.007 | 0.84 | 0.023 | 0.023 |
| coefficient of variation (%) | 0.92 | 0.67 | 1.47 | 0.017 | 1.17 | 1.19 |

From the above results, it has been confirmed that accuracy of measurement including reproducibility and correctness is improved in the automated analyzers employing non-contact type stirring mechanisms with the use of the measuring reagent of the present invention containing a silicone-based defoaming agent as compared with conventional measuring reagents.

### INDUSTRIAL APPLICABILITY

The present invention has made it possible to provide an agglutination measuring reagent for automated analysis based on a homogeneous measurement method employing insoluble carrier particles which enables highly accurate measurement without being affected by the matrix effect originating from the sample and regardless of the specifications of the automated analyzer.

## Claims

1. A measuring reagent for a homogeneous latex agglutination immunoassay method for an automated analyzer comprising latex particles, **characterized in that**
the measuring reagent comprises more than one constituent reagents,
one of the constituent reagents contains the latex particles,
the latex particles support a substance that binds an analyte with a high affinity, the substance being selected from a polyclonal antibody, a monoclonal antibody, a recombinant antibody, a functional fragment of the previously mentioned antibodies, or a natural or recombinant antigen,
another constituent reagent contains a silicone-based defoaming agent, wherein a concentration of the silicone-based defoaming agent in said another constituent reagent is 0.0001 to 0.1 %,
the protein concentration of each constituent reagent is less than 2% (w/v), and said measuring reagent is used for a measurement wherein a total liquid volume of a sample and said measuring reagent dispensed by the automated analyzer is less than 300 µL, a liquid volume of said constituent reagent comprising the latex particles accounts for 20 to 50% (v/v) relative to the total liquid volume,
wherein the protein concentrations of the constituent reagents do not include proteins supported on the carrier particles as the substance that binds the analyte with high affinity, or blocking proteins that coat the carrier particles.

2. The reagent according to claim 1, wherein the silicone-based defoaming agent is of a type selected from the group consisting of oil, oil compound, solution, self-emulsion, emulsion, and a mixture thereof.

3. A homogeneous latex agglutination immunoassay method using an automated analyzer comprising the steps of:
1) dispensing a measuring reagent and a sample containing an analyte, wherein
the measuring reagent comprises more than one constituent reagents;
one of the constituent reagents contains latex particles;
the latex particles support a substance that binds an analyte with a high affinity, the substance being selected from a polyclonal antibody, a monoclonal antibody, a recombinant antibody, a functional fragment of the previously mentioned antibodies, or a natural or recombinant antigen;
another constituent reagent contains a silicone-based defoaming agent, wherein a concentration of the silicone-based defoaming agent in said another constituent reagent is 0.0001 to 0.1%; and
the protein concentration in each constituent reagent is less than 2% (w/v);
2) mixing the sample and the measuring reagent in such a way that a total liquid volume of the sample and the measuring reagent dispensed by the automated analyzer is less than 300 µL, said
constituent reagent containing the latex particles accounts for 20 to 50% (v/v) relative to the total liquid volume, the final protein concentration during the measurement in the automated analyzer is less than 1% (w/v) relative to the total liquid volume, wherein the protein concentrations of the constituent reagents and the final protein concentration do not include proteins supported on the carrier particles as the substance that binds the analyte with high affinity, or blocking proteins that coat the carrier particles; and
3) detecting the analyte.

## Patentansprüche

1. Messreagenz für ein homogenes Latex-Agglutinations-Immunoassayverfahren für einen automatisierten Analysator, umfassend Latexpartikel, **dadurch gekennzeichnet, dass**
das Messreagenz mehr als ein Bestandteilreagenz umfasst,
eines der Bestandteilreagenzien Latexpartikel enthält,
die Latexpartikel eine Substanz tragen, die einen Analyten mit hoher Affinität bindet, wobei die Substanz aus einem polyklonalen Antikörper, einem monoklonalen Antikörper, einem rekombinanten Antikörper, einem funktionellen Fragment der zuvor genannten Antikörper oder einem natürlichen oder rekombinanten Antigen ausgewählt ist,
ein anderes Bestandteilreagenz ein silikonbasiertes Entschäumungsmittel enthält, wobei die Konzentration des silikonbasierten Entschäumungsmittels in dem anderen Bestandteilreagenz 0,0001 bis 0,1 % beträgt,
die Proteinkonzentration von jedem Bestandteilreagenz weniger als 2 % (Gew./Vol.) ist, und
das Messreagenz für eine Messung verwendet wird, bei der das gesamte Flüssigkeitsvolumen einer Probe und des Messreagenzes, das durch den automatisierten Analysator dispensiert wird, weniger als 300 µl beträgt, ein Flüssigkeitsvolumen des Bestandteilreagenzes, das den Latexpartikel umfasst, 20 bis 50 Vol.%, bezogen auf das gesamte Flüssigkeitsvolumen, ausmacht,
wobei die Proteinkonzentrationen der Bestandteilreagenzien nicht Proteine, die von den Trägerpartikeln als die Substanz, die mit hoher Affinität den Analyten bindet, getragen werden, oder blockierende Proteine, die die Trägerpartikel überziehen, mit einschließen.

2. Reagenz gemäß Anspruch 1, wobei das silikonbasierte Entschäumungsmittel von einem Typ ist, der aus der Gruppe ausgewählt ist, die aus einem Öl, einer Ölverbindung, einer Lösung, einer Selbstemulsion, einer Emulsion und einer Mischung davon besteht.

3. Homogenes Latex-Agglutinations-Immunoassayverfahren unter Verwendung eines automatisierten Analysators, umfassend die Schritte:
1) das Dispensieren eines Messreagenzes und einer Probe, enthaltend einen Analyten, wobei
das Messreagenz mehr als ein Bestandteilreagenz umfasst;
eines der Bestandteilreagenzien Latexpartikel enthält;
die Latexpartikel eine Substanz tragen, die mit hoher Affinität einen Analyten bindet, wobei die Substanz aus einem polyklonalen Antikörper, einem monoklonalen Antikörper, einem rekombinanten Antikörper, einem funktionellen Fragment der zuvor genannten Antikörper oder einem natürlichen oder rekombinanten Antigen ausgewählt ist;
ein anderes Bestandteilreagenz ein silikonbasiertes Entschäumungsmittel enthält, wobei die Konzentration des silikonbasierten Entschäumungsmittels in dem anderen Bestandteilreagenz 0,0001 bis 0,1 % beträgt; und
die Proteinkonzentration in jedem Bestandteilreagenz weniger als 2 % (Gew./Vol.) beträgt;
2) das Mischen der Probe und des Messreagenzes auf eine Art, dass das gesamte Flüssigkeitsvolumen der Probe und des Messreagenzes, das durch den automatisierten Analysator dispensiert wird, weniger als 300 µl beträgt, wobei das Bestandteilreagenz, das die Latexpartikel enthält, 20 bis 50 Vol.%, bezogen auf das gesamte Flüssigkeitsvolumen, ausmacht, die finale Proteinkonzentration während der Messung in dem automatisierten Analysator weniger als 1 % (Gew./Vol.), bezogen auf das gesamte Flüssigkeitsvolumen, ist, wobei die Proteinkonzentrationen der Bestandteilreagenzien und die finale Proteinkonzentration nicht Proteine, die von den Trägerpartikeln als Substanz, die mit hoher Affinität den Analyten bindet, getragen werden, oder blockierende Proteine, die die Trägerpartikel überziehen, mit einschließen; und
3) das Detektieren des Analyten.

## Revendications

1. Réactif de mesure pour un procédé de dosage immunologique d'agglutination au latex homogène pour un analyseur automatisé comprenant des particules de latex, **caractérisé en ce que**
le réactif de mesure comprend plus d'un réactif constitutif,
un des réactifs constitutifs contient les particules de latex,
les particules de latex supportent une substance qui se lie à un analyte avec une affinité élevée, la substance étant sélectionnée parmi un anticorps polyclonal, un anticorps monoclonal, un anticorps recombinant, un fragment fonctionnel des anticorps mentionnés précédemment, ou un antigène naturel ou recombinant, un autre réactif constitutif contient un agent antimousse à base de silicone, dans lequel une concentration en agent antimousse à base de silicone dans ledit autre réactif constitutif est 0,0001 à 0,1 %,
la concentration en protéines de chaque réactif constitutif est inférieure à 2 % (p/v), et
ledit réactif de mesure est utilisé pour une mesure dans laquelle un volume de liquide total d'un échantillon et dudit réactif de mesure distribué par l'analyseur automatisé est inférieur à 300 µL, un volume de liquide dudit réactif constitutif comprenant les particules de latex compte pour 20 à 50 % (v/v) par rapport au volume de liquide total,
dans lequel les concentrations en protéines des réactifs constitutifs n'incluent pas de protéines supportées sur les particules porteuses comme la substance qui se lie à l'analyte avec une affinité élevée, ou des protéines bloquantes qui recouvrent les particules porteuses.

2. Réactif selon la revendication 1, dans lequel l'agent antimousse à base de silicone est d'un type sélectionné dans le groupe constitué d'une huile, d'un composé huileux, d'une solution, d'une auto-émulsion, d'une émulsion, et d'un mélange de ceux-ci.

3. Procédé de dosage immunologique d'agglutination au latex homogène utilisant un analyseur automatisé comprenant les étapes consistant à :
1) distribuer un réactif de mesure et un échantillon contenant un analyte, dans lequel le réactif de mesure comprend plus d'un réactif constitutif;
un des réactifs constitutifs contient des particules de latex;
les particules de latex supportent une substance qui se lie à un analyte avec une affinité élevée, la substance étant sélectionnée parmi un anticorps polyclonal, un anticorps monoclonal, un anticorps recombinant, un fragment fonctionnel des anticorps mentionnés précédemment, ou un antigène naturel ou recombinant;
un autre réactif constitutif contient un agent antimousse à base de silicone, dans lequel une concentration d'agent antimousse à base de silicone dans ledit autre réactif constitutif est de 0,0001 à 0,1 %; et
la concentration en protéines dans chaque réactif constitutif est inférieure à 2% (p/v) ;
2) mélanger l'échantillon et le réactif de mesure de telle sorte qu'un volume de liquide total de l'échantillon et du réactif de mesure distribué par l'analyseur automatisé est inférieur à 300 µL, ledit réactif constitutif contenant des particules de latex compte pour 20 à 50 % (v/v) par rapport au volume de liquide total, la concentration en protéines finale pendant la mesure dans l'analyseur automatisé est inférieure à 1 % (p/v) par rapport au volume de liquide total, dans lequel les concentrations en protéines des réactifs constitutifs et la concentration en protéines finale n'incluent pas de protéines supportées sur des particules porteuses comme la substance qui se lie à l'analyte avec une affinité élevée, ou des protéines bloquantes qui recouvrent les particules porteuses ; et
3) détecter l'analyte.
